(19) Europäisches Patentamt
European Patent Office
Office européen des brevets

(11) Publication number: 0 343 772 A2

# (12) EUROPEAN PATENT APPLICATION

(21) Application number: 89302526.2

(51) Int. Cl.[4]: C07F 15/00

(22) Date of filing: 15.03.89

(30) Priority: 26.05.88 JP 129376/88

(43) Date of publication of application: 29.11.89 Bulletin 89/48

(84) Designated Contracting States: CH DE FR GB LI SE

(71) Applicant: AGENCY OF INDUSTRIAL SCIENCE AND TECHNOLOGY
3-1, 1-chome, Kasumigaseki
Chiyoda-ku Tokyo(JP)

(72) Inventor: Okuno, Hiroaki
670-101, Namiki 3
Tsukuba-shi Ibaraki-ken(JP)
Inventor: Shimura, Takehiko
306, 4-29, Ikenodai 1-chome Taito-ku
Tokyo(JP)
Inventor: Tomohiro, Takenori
401-608, Azuma 1
Tsukuba-shi Ibaraki-ken(JP)

(74) Representative: Miller, Joseph et al
J. MILLER & CO. Lincoln House 296-302 High Holborn
London WC1V 7JH(GB)

## (54) Process for preparing platinum green complex.

(57) A process for preparing a platinum green complex which comprises reacting a compound represented by the following general formula (I) with a compound selected from among uridine, thymidine, uracil, thymine, 2′-deoxyuridine, uridine-5′-monophosphate, 5-fluorouracil and 1,2-diaminocyclohexane in the presence of a silver salt and hydrogen peroxide in a solvent:

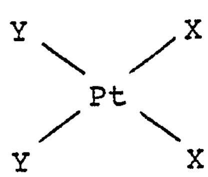

(I)

wherein X represents Cl or I; and Y is a group selected from among $NH_3$, $C_nH_{2n+1}NH_2$, $NH_2$-$C_mH_{2m}$-$NH_2$ and 1,2-diaminocyclohexane, wherein n is an integer of 1 to 12 while m is an integer of 2 to 10.

EP 0 343 772 A2

# PROCESS FOR PREPARING PLATINUM GREEN COMPLEX

This invention relates to a process for selectively preparing a platinum green complex which has an anti-tumor activity and is, therefore, highly useful as a pharmaceutical.

The excellent anti-tumor activity of cisplatin, which was proved by Rosenberg et al [cf. Nature, 222, 385 (1969)], has made this compound widely useful in the treatment of, for example, prostatic cancer, ovarian cancer and gastric cancer. However cisplatin also exerts some side effects including renal toxicity, vomiturition and vomiting, which brings about serious problems. Thus there has been attempted to improve this compound to thereby relieve these side effects. In addition, there has been frequently attempted to synthesize a platinum complex which has an anti-tumor activity higher than that of cisplatin and yet shows little side effects.

U.S. Patent No. 4,419,351 teaches that a platinum-[2,4-dioxopyrimidine compound] blue or green complex or a mixture thereof, each having anti-tumor, anti-bacterial and anti-viral effects, can be obtained by reacting 2,4-dioxopyrimidine compound with cis-diaquodiammineplatinum (II).

Furthermore the structure of a green complex of cis-diamminedichloroplatinum (II) and α-pyrrolidone is reported [cf. J. Am. Chem. Soc., 106, 2049 - 2054 (1984)].

According to the U.S. Patent No. 4,419,351 cited above, platinum complexes with bases such as uracil, thymine, pyrimidine or polyuracil, uridine derivatives or thymidine derivatives are obtained. In the case of the platinum complex with these bases, a platinum blue complex is obtained as the major component while a platinum green complex is obtained as the second precipitate. The platinum blue complex is identified by, for example, elemental analysis and visible absorption spectrometry and further discussed from a biological viewpoint.

However the physicochemical properties of the platinum green complexes have never been clarified yet. Further, there have never been reported the purification, physicochemical properties and anti-tumor activities of water soluble platinum green complexes which would not form precipitate. On the other hand, platinum complexes with uridine derivatives or thymidine derivatives are limited to blue complexes and the description on only the anti-tumor activities thereof is found at best. That is, there is any description regarding neither the preparation nor antitumor activities of green complex of uridine or thymidine derivatives.

It is an object of the present invention to efficiently prepare a platinum green complex which is useful as an anti-tumor agent.

The process for preparing a platinum green complex of the present invention comprises reacting a compound represented by the following general formula (I) with a compound selected from among uridine, thymidine, uracil, thymine, 2′-deoxyuridine, uridine-5′-monophosphate, 5-fluorouracil and 1,2-diaminocyclohexane in the presence of a silver salt and hydrogen peroxide:

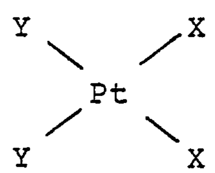

(I)

In the above formula (I), X is Cl or I, while Y is a group selected from among $NH_3$, $C_nH_{2n+1}NH_2$, $NH_2\text{-}C_mH_{2m}\text{-}NH_2$ and

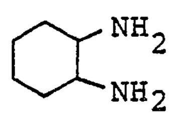

wherein n is an integer of 1 to 12 while m is an integer of 2 to 10.

The platinum green complex obtained by the process of the present invention is one wherein a compound selected from among uridine, thymidine, uracil, thymine, 2′-deoxyuridine, uridine-5′-monophosphate, 5-fluorouracil and 1,2-diaminocyclohexane, which will be called a substrate hereinafter, is coordinated to platinum. The platinum green complex carries 3 to 18 platinum atoms per molecule and a particularly high anti-tumor activity can be obtained when it carries 6 to 15 platinum atoms per molecule.

The reaction between the compound of the above general formula (I) and the above mentioned substrate may be carried out in a solvent at a pH value of 1 to 8, preferably 2 to 6, at a temperature of 0 to 100° C, preferably 1 to 75° C, for 30 minutes to two months, preferably 45 minutes to one month.

Examples of the solvent include water, alcohols such as methanol, ethanol and tert-butanol, ethers such as dimethoxyethane, tetrahydrofuran and dioxane, dimethylformamide, dimethyl sulfoxide, hexamethylenephosphoramide, acetic acid, pyridine and mixtures thereof.

The compound of the general formula (I) and the above substrate may be used at a molar ratio of 1 : 1 to 1: 4, generally around 1 : 1.

Any silver salt may be used in the present invention so long as it can react with the compound of the general formula (I) to thereby give silver chloride. Examples thereof include $Ag_2SO_4$ , $AgNO_3$ and $AgClO_4$. Among these compounds, $Ag_2SO_4$ is the most preferable.

The hydrogen peroxide to be used in the present invention is preferably in the form of a 0.1 to 10% aqueous solution of hydrogen peroxide.

The compound of the general formula (I) may be prepared, for example, in the following manner. To potassium tetrachloroplatinate (II) ($K_2PtCl_4$) is added potassium iodide (KI) in an amount four times as much as the former. The obtained mixture is heated at approximately 50° C for 15 to 45 minutes. When the reaction mixture turns dark red, aqueous ammonia in an amount 10 to 20 times as much as that of the reaction mixture is added thereto. Thus a yellow powder precipitates. This yellow powder is filtered, washed with ethanol and ether and then dried to thereby give the aimed

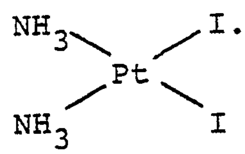

The platinum green complex thus obtained may be isolated and purified in a conventional manner. Namely, the reaction mixture may be separated by, for example, extraction with a solvent, precipitation and ion exchange, or various chromatographic procedures such as partition chromatography, gel filtration chromatography or affinity chromatography.

Gel filtration chromatography is particularly preferable in the process of the present invention. Any carrier may be employed therefor and examples thereof include Sephadex, Sephacryl, Sepharose, Biogel, Toyopearl, Fractogel and Cellulofine. It is preferable to carry out these isolation and purification procedures under anaerobic conditions, such as under a nitrogen gas stream.

The platinum green complex thus isolated may be identified by a visible absorption spectrum characteristic thereto. For example, a platinum green complex wherein uridine is a ligand shows a characteristic absorption at 730 nm and an intense $\pi \rightarrow \pi^*$ absorption at 260 to 270 nm, i.e., within the UV region.

The IR spectrum of this complex shows a C=O stretching vibration shifted to 1645 $cm^{-1}$, compared with that of uridine observed at 1690 $cm^{-1}$. The $^{13}C$-NMR spectrum thereof shows remarkably large shifts toward a lower magnetic field side corresponding to C-4 (11 to 8 ppm) and C-2 (6 to 4 ppm). These facts strongly suggest that the C-4 or C-2 position of the uridine is coordinated. This green complex shows a paramagnetic resonance spectrum (ESR) or Pt (III). This spectrum shows a typical axially symmetric pattern ($g_\parallel$ = 1.991, $g_\perp$ = 2,413). Examination from the viewpoint of a hyperfine structure has revealed that unpaired electrons are not localized in a single platinum atom but are delocalized over a plurality of platinum atoms.

Various platinum green complexes are prepared by reacting a compound of the general formula (I) wherein Y is an $NH_3$ molecule while X is an iodine atom, with uridine under various conditions. Table 1 shows the results.

Table 1

Synthesis of platinum-uridine green complex

| Platinum green complex | Temp. (° C) | Reagent a) | Concn. (M) b) | Time | Yield (%) c) |
|---|---|---|---|---|---|
| (1) | 2 | A | 0.1 | 2wk | 43.8 |
| (2) | 2 | A | 0.1 | 4wk | 51.0 |
| (3) | 25 | A | 0.1 | 24hr | 46.1 |
| (4) | 40 | A | 0.1 | 3hr | 33.4 |
| (5) | 75 | A | 0.01 | 3hr | 61.0 |
| (6) | 75 | A | 0.1 | 45min | 74.4 |
| (7) (blue) | 60 | B | 0.1 | 2hr | 40.7 |

Note: a) A: $Ag_2SO_4$ and 1% aqueous solution of hydrogen peroxide used.

B: oxidized with air.

b) substrate concentration.

c) tetranuclear platinum complex, calculated by assuming the average valence of platinum to be 2.25.

The results of the elemental analysis of these platinum green complexes are as follows.

Platinum green complex (1):

Found: C, 15.47; H, 3.29; N, 9.31; and Pt *, 42.6

Platinum green complex (2):

Found: C, 15.39; H, 3.25; N, 9.78; and Pt, 41.8

Platinum green complex (3):

Found: C, 16.74; H, 3.11; N, 9.37; and Pt, 40.5

Platinum green complex (4):

Found: C, 9.67; H, 2.72; N, 9.29; and Pt, 47.7

Platinum green complex (5):

Found: C, 16.31; H, 2.68; N, 9.35; and Pt, 40.7

Platinum green complex (6):

Found: C, 15.37; H, 3.09; N, 9.38; and Pt, 41.5

*: calculated based on the ash content, the same will apply hereinbelow.

Platinum green complex (7) (blue):
Found: C, 20.55; H, 3.02; N, 8.20; and Pt, 38.0

Similarly, the compound of the general formula (I) (Y = $NH_3$ and X = I) is reacted with each substrate shown in Table 2. Table 2 also shows the reaction conditions and the results of elemental analysis.

Table 2

| Synthesis of platinum green complex [a] | | | | |
|---|---|---|---|---|
| Platinum green complex | Substrate | Temp. (° C) | Time | Yield [b] (%) |
| (8) | uracil | 37 | 3h | 38.8 |
| (9) | 5-fluorouracil | 75 | 45min | 34.0 |
| (10) | diaminocyclohexane [c] | 50 | 1d | 69.3 |
| (11) | guanosine [d] | 75 | 2d | 47.9 |
| (12) | inosine [e] | 75 | 2d | 94.3 |
| (13) | uridine-5′-monophosphate | 25 | 1d | 13.9 |
| (14) | thymidine | 75 | 30min | 22.4 |

Note:

a) synthesized by using 0.1 M of the substrate and 1% $H_2O_2$
b) tetranuclear platinum complex, calculated by assuming the average valence of platinum to be 2.25
c) yellow complex
d) yellow complex
e) colorless complex

Elemental analysis (found):

Platinum green complex (8):
Found: C, 3.37; H, 2.03; N, 9.15; and Pt, 58.2
Platinum green complex (9):
Found: C, 6.09; H, 2.22; N, 10.84; and Pt, 52.9
Platinum green complex (10):
Found: C, 24.23; H, 4.11; N, 8.05; and Pt, 36.6
Platinum green complex (11):
Found: C, 19.94; H, 3.29; N, 16.06; and Pt, 32.1
Platinum green complex (12):
Found: C, 19.32; H, 3.00; N, 14.12; and Pt, 29.2
Platinum green complex (13):
Found: C, 14.18; H, 2.89; N, 8.86; and Pt, 49.4
Platinum green complex (14):
Found: C, 10.81; H, 2.81; N, 8.51; and Pt, 48.1

As shown in the following Test Example, each platinum green complex obtained by the process of the present invention has a high anti-tumor activity which makes it effective as a carcinostatic agent.

Test Example: Anti-tumor activity on L1210

The anti-tumor activity of each platinum green complex of the present invention was evaluated by determining its growth inhibition effect on cultured L1210 cells. Namely, a test sample (final concentration: 5 μg/ml or 10 μg/ml) was injected into a suspension of a concentration of 1 x $10^5$ cells/ml. These cells were cultured at 37° C usually for four days. A GIBCO RPMI 1640 medium containing kanamycin and 10% of

fetal bovin serum (FBS) was employed in the culture. The inhibition ratio was determined by comparing the growth of the test culture with that of a control which generally showed a concentration of $1.4 \times 10^6$ cells/ml. Table 3 shows the results.

Table 3

| Physiological activity of platinum green complex [a] | | |
|---|---|---|
| Compound | Growth inhibition ratio (%) | |
| | 1 µg/ml [b] | 10 µg/ml [b] |
| (1) | 59.1 | 99.8 |
| (2) | 55.8 | 99.4 |
| (3) | 63.7 | 99.1 |
| (4) | 80.2 | 99.7 |
| (5) | 26.4 | 98.1 |
| (6) | 25.5 | 99.1 |
| (7) | - | -1.9 |
| (8) | 86.3 | 100 |
| (9) | 46.4 | 99.7 |
| (10) | 31.5 | 97.8 |
| (11) | 7.1 | 20.4 |
| (12) | -0.7 | 1.9 |
| (13) | 12.5 | 99.0 |
| (14) | 58.3 | 100 |

Note:

a) Cultured L1210 cells are used.
b) Concentration of a sample used in the evaluation of the activity

Table 3 indicates that platinum green complexes exert high activities on L1210 cells while platinum blue complexes show no activity thereon, remarkably different from the former ones. Detailed examination further suggests that the physiological activity of a platinum green complex correlates to the molecular size of the same. Namely, the activity would increase with a decrease in molecular size. Specifically, those carrying 3 to 18, in particular, 6 to 15 platinum atoms per molecule are highly active.

Giant cells having a diameter approximately twice as large as that of common L1210 cells were observed. It was found that the ratio of these giant cells would increase with an increase in the anti-tumor activity of the platinum green complex.

The compound obtained by the process of the present invention may be formulated into pharmaceuticals, for example, tablets, granules, powder, capsules, injection, ointment or suppositories, by mixing it with pharmaceutically acceptable additives such as excipients, carriers or diluents. It may be either orally or parenterally administered. The dose of this compound may vary depending on, for example, the type and condition of the tumor to be treated, the administration way and the age and body weight of the patient. Generally speaking, it may be administered in a dose of 0.1 to 5,000 mg/kg/day, preferably 0.1 to 1,000 mg/kg/day and still preferably 1 to 500 mg/kg/day, once to six times a day.

Example:

To 145 mg of cis-diiododiammineplatinum (II) and 93.5 mg of silver sulfate were added 3 ml of water and 73.2 mg (0.03 mmol) of uridine. Further 336 µl of a 1% aqueous solution of hydrogen peroxide was added thereto. The resulting mixture was allowed to react by stirring at 40° C under an argon gas stream for three hours. The obtained reaction mixture was filtered and the filtrate was purified by gel filtration chromatography wherein Toyopearl was used as a carrier. Thus 53.4 mg of a platinum green complex

having uridine as a ligand was obtained. The above procedure was carried out in a nitrogen or argon atmosphere.

Visible region absorption: λmax 730 nm
IR (KBr): 1645 (C=0), 870, 815 and 585 (Pt-N) $cm^{-1}$
m.p.: >300° C
Circular dichroism (CD spectrum):
[θ] (λmax nm); 37,800 (272), -37,800 (219)
$^{13}C$-NMR ($D_2O$; DDS standard): ppm
63.5, 72.1, 76.3, 86.9, 92.8, 104.7, 143.6, 160.2, 158.5, 179.8 and 176.7

## Claims

1. A process for preparing a platinum green complex which comprises reacting a compound represented by the following general formula (I) with a compound selected from among uridine, thymidine, uracil, thymine, 2′-deoxyuridine, uridine-5′-monophosphate, 5-fluorouracil and 1,2-diaminocyclohexane in the presence of a silver salt and hydrogen peroxide in a solvent:

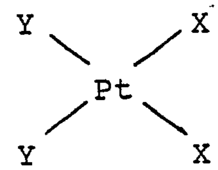

(I)

wherein X represents Cl or I; and Y is a group selected from among $NH_3$, $C_nH_{2n+1}NH_2$, $NH_2$ $C_mH_{2m}$-$NH_2$ and 1,2-diaminocyclohexane, wherein n is an integer of 1 to 12 while m is an integer of 2 to 10.

2. A process for preparing a platinum green complex as set forth in claim 1, wherein said platinum green complex carries 3 to 18 platinum atoms per molecule.

3. A process for preparing a platinum green complex as set forth in claim 1, wherein said platinum green complex carries 6 to 15 platinum atoms per molecule.

4. A process for preparing a platinum green complex as set forth in claim 1, wherein said solvent comprises at least one compound selected from among water, alcohols, ethers, dimethylformamide, dimethyl sulfoxide, hexamethylenephosphoramide, acetic acid and pyridine.

5. A process for preparing a platinum green complex as set forth in claim 1, wherein said compound of the general formula (I) and said compound are used at a molar ratio of 1 : 1 to 1 : 4.

6. A process for preparing a platinum green complex as set forth in claim 1, wherein said silver salt is a compound selected from among $Ag_2SO_4$, $AgNO_3$ and $AgClO_4$.

7. A process for preparing a platinum green complex as set forth in claim 1, wherein said hydrogen peroxide is in the form of a 0.1 to 10% aqueous solution of hydrogen peroxide.